# EUROPEAN PATENT SPECIFICATION

(11) **EP 2 575 680 B1**
(45) Date of publication and mention of the grant of the patent: **22.04.2015**
(21) Application number: 11789355.2
(22) Date of filing: 16.05.2011
(51) Int. Cl.: A61F 2/16

(54) **PUPIL CENTERED FOVEA FOCUSED OPTICS ASSEMBLY FOR INTRAOCULAR LENS**
PUPILLENZENTRIERTE UND FOVEA-FOKUSSIERTE OPTIKANORDNUNG FÜR KONTAKTLINSEN
ENSEMBLE OPTIQUE CONCENTRÉ SUR UNE FOVÉA ET CENTRÉ SUR UNE PUPILLE POUR UNE LENTILLE INTRAOCULAIRE

(30) Priority: 31.05.2010 IN CH14892010
(43) Date of publication of application: 10.04.2013
(73) Proprietor: Mirlay, Ram Srikanth, Bangalore 560 080 Karnataka (IN)
(72) Inventor: Mirlay, Ram Srikanth, Bangalore 560 080 Karnataka (IN)
(74) Representative: Papa, Elisabetta
(86) International application number: PCT/IN2011/000340
(87) International publication number: WO 2011/151839

(56) References cited:
- EP-A2- 0 949 529
- CN-A- 1 087 505
- CN-A- 101 534 749
- US-A- 4 441 217
- US-A- 4 441 217
- US-A- 5 652 640
- US-A1- 2006 184 244
- US-A1- 2006 184 244
- US-A1- 2008 269 881
- ASANO-KATO ET AL: "Pupil decentration and iris tilting detected by Orbscan: Anatomic variations among healthy subjects and influence on outcomes of laser refractive surgeries", JOURNAL CATARACT AND REFRACTIVE SURGERY, SURGERY, FAIRFAX, VA, US, vol. 31, no. 10, 1 October 2005 (2005-10-01), pages 1938-1942, XP005204785, ISSN: 0886-3350, DOI: 10.1016/J.JCRS.2005.03.073

## Description

### Technical field

The embodiments herein generally relate to the field of ophthalmology and particularly to an optical lens used in ophthalmology, for example, an implantable intraocular lens to be affixed in a mammalian eye. The embodiments herein more particularly relate to a pupil-centered and fovea-focused optics assembly for an implantable intraocular lens.

### Description of the Related Art

Intraocular lenses (IOLs) are used in cataract eye surgery to replace the human/opacified mammalian (cataractous) lens for cataractous or refractive/or other reasons. A pupil of the mammalian eyes is not always centered to the visual axis of the eye. There is a normal, natural and common decentering of the pupil with respect to the optics of the eye. After surgical implementation of the IOL, it is often found that the center of the IOL optics does not coincide with the center of the pupil or with the visual axis. IOL. This results in a compromised sharpness of the vision (visual acuity or activity) and visual quality.

The normal human (mammalian) eye, seen as an optical device, has certain design defects, which limit its potential maximum vision. These are given as follows. First, the center of the pupil does not always exactly coincide with the center of the lens (natural or surgically implanted IOL) thereby causing an intrinsic reduction in the image quality on the retina. The de-centering can be up to 200 microns. Secondly, the optical axis of the human lens or the IOL when implanted does not fall exactly on Fovea Centralis, the most sensitive and 'seeing' (visual) point in the retina. It may be "off the axis" by a maximum of 7 degrees.

These two natural design defects exist in all the human eyes in varying degrees. These are well-known medical facts for decades and are referred to as angle kappa, angle gamma, de-centered pupil, tilt of the natural lens etc.

When lenses opacify due to age or other reasons, cataract surgery is performed and a new artificial lens (IOL or Intra Ocular Lens) is implanted in the same place (Capsular BAG) as the original lens. Therefore, the IOL (prior art) too continues to inherit and cause the same pre-existing defects of mis-aligned optics (de-centered optics), i.e., the center of the IOL does not fall on/coincide with the center of the pupil and the optical axis of the IOL does not fall exactly on the Fovea, in most human/mammalian eyes.

At present, there is no system and method to align the geometric centre of the lens with the visual axis of the pupil and to focus (or point) the implanted lens towards the fovea, to improve the visual activity and the visual quality. Hence, there is a need for a new system and method to align the optical centre of the implanted lens with the visual axis of the pupil and to tilt the implanted lens to focus towards the fovea.

US 4,441,217 discloses an intraocular lens structure having a light focusing lens body and oppositely disposed support members for supporting the lens body upon implantation in the eye. Such support members position the optical axis of the lens body at a point which is offset from the geometric center axis of the cornea.

US 5,652,640 discloses methods for evaluating vision through an intraocular lens. The lens can be moved, tilted or decentered relative to the cornea or fovea window.

US 2008/269881 discloses intraocular lenses allowing a centration of optics and pupil that can be adjusted by haptics having offset regions.

### OBJECTS OF THE EMBODIMENTS

A primary objective of the embodiments herein is to develop an optics assembly to align the optical centre of the implanted lens with the center (optical axis or visual axis) of the pupil to correct an aberration.

Another objective of the embodiments herein is to develop an optics assembly to tilt the implanted lens to focus (point) towards the fovea to improve the sharpness and the quality of the vision (more towards the retinal capacity limit).

These and the other objects and advantages of this invention will be understood easily by studying the following specification with the accompanying drawings.

### SUMMARY

Various embodiments herein provide a pupil-centered and fovea-focused optics assembly for an implantable intraocular lens. The assembly comprises a ring platform provided inside a capsular bag of a mammalian eye and an intraocular lens with an optical center and a geometric center mounted on the ring platform. The optical center of the intraocular lens is calculatedly de-centered with respect to the geometric center of the intraocular lens to align the optical centre of the lens with a visual axis of a pupil of the mammalian eye, to improve the visual quality and to prevent an aberration.

According to one embodiment herein, a plane of the intraocular lens is turned and tilted through a preset angle to point an optic axis of the intraocular lens to the Fovea and the optical centre of the intraocular lens is decentered from the geometric centre of the intraocular lens to compensate for the de-centered pupil.

According to one embodiment herein, the preset angle is calculated based on an overall diameter of the capsular bag, a thickness of the ring platform, a decentering direction and a decentering distance of the optical axis of the intraocular lens.

According to one embodiment herein, the overall diameter of the capsular bag, the thickness of the ring platform, the decentering direction and the decentering distance of the optical axis of the intraocular lens are calculated by performing a three dimensional scanning of the mammalian eye.

According to one embodiment herein, the intraocular lens is mounted on the ring platform through a plurality of haptics. The plurality of haptics holds the intraocular lens in a desired place.

According to one embodiment herein, the ring platform arranged inside the capsular bag supports the intraocular lens through the haptics and points the intraocular lens towards a fovea of the mammalian eye.

According to one embodiment herein, the haptics of the intraocular lens is fixed to a front part or a middle part or a back part of the ring platform. When an upper haptics of the intraocular lens is fixed to the front part of the ring platform, then a lower haptics is fixed to the back part of the ring platform.

According to one embodiment herein, the ring platform is provided with markings to identify a vertical meridian and a horizontal meridian. Similarly, the intraocular lens and the haptics are provided with the markings to identify a vertical meridian and a horizontal meridian.

According to one embodiment herein, the ring platform, the optic and the haptics are made of bio compatible material with the required rigidity, flexibility and optical qualities. The ring platform has an over-all diameter of 11 to 14 mm for humans and it can be varied for veterinary use depending on the species. The thickness of the ring platform will vary from 1mm to 2.5 mm. It may be of hydrophobic or hydrophilic material. It may be coated with or stored/loaded with medications such as steroids, antibiotics, anti-glaucoma drugs, anti-degenerative drugs, anti-mitotic agents to reduce posterior capsular opacification, etc.

According to one embodiment herein, the size of the haptics is in the range of 0.5 mm - 3.5 mm. The shape can be of any stable one but the number of haptics has to be 4 or more to provide stability against rolling.

According to one embodiment herein, the optic size is varied from 5 mm to 10 mm or more, based on the mammalian eye. The optic design too, can be of any functional type. It is also applicable to aspheric, multifocal, accommodative, and all types of intraocular lenses.

### BRIEF DESCRIPTION OF THE DRAWINGS

The other objects, features and advantages will occur to those skilled in the art from the following description of the preferred embodiment and the accompanying drawings in which:
FIG. 1 illustrates the front view of a pupil-centered fovea focused intraocular lens, according to one embodiment herein.
FIG. 2 illustrates the side view of a pupil-centered fovea-focused intraocular lens, according to one embodiment herein.

Although the specific features of the embodiments herein are shown in some drawings and not in others. This is done for convenience only as each feature may be combined with any or all of the other features in accordance with the embodiments herein.

### DETAILED DESCRIPTION OF THE EMBODIMENTS HEREIN

In the following detailed description, a reference is made to the accompanying drawings that form a part hereof, and in which the specific embodiments that may be practiced is shown by way of illustration. These embodiments herein are described in sufficient detail to enable those skilled in the art to practice the embodiments herein and it is to be understood that the logical, mechanical and other changes may be made without departing from the scope of the embodiments herein. The following detailed description is therefore not to be taken in a limiting sense.

A pupil-centered and fovea-focused optics assembly for an implantable intraocular lens comprises a ring platform provided inside a capsular bag of a mammalian eye and an intraocular lens with an optical centre and a geometric centre is mounted on the ring platform. The optical centre of the intraocular lens is calculatedly de-centered with respect to the geometric centre of the intraocular lens to align the optical centre of the lens with a visual axis of pupil of the mammalian eye, to improve the visual quality and to prevent an aberration.

According to one embodiment herein, a plane of the intraocular lens is turned and tilted through a preset angle to point an optic axis of the intraocular lens to the fovea and the optical centre of the intraocular lens is de-centered from the geometric centre of the intraocular lens to compensate for the de-centered pupil.

According to one embodiment herein, the preset angle is calculated based on an overall diameter of the capsular bag, a thickness of the ring platform, a decentering direction and a decentering distance of the optical axis of the intraocular lens.

According to one embodiment herein, the overall diameter of the capsular bag, the thickness of the ring platform, the decentering direction and the decentering distance of the optical axis of the intraocular lens are calculated by performing a three dimensional scanning of the mammalian eye.

According to one embodiment herein, the intraocular lens is mounted on the ring platform through a plurality of haptics. The plurality of haptics holds the intraocular lens in a desired place.

According to one embodiment herein, the ring platform arranged inside the capsular bag supports the intraocular lens through the haptics and points the intraocular lens towards a Fovea of the mammalian eye.

According to one embodiment herein, the haptics of the intraocular lens is fixed to a front part or a middle part or a back part of the ring platform. If upper haptics of the intraocular lens are fixed to the front part of the ring platform, then lower haptics are fixed to the back part of the ring platform.

According to one embodiment herein, the ring platform is provided with markings to identify a vertical meridian and a horizontal meridian. Similarly, the intraocular lens and the haptics are provided with the markings, to identify a vertical meridian and a horizontal meridian.

According to one embodiment herein, the ring platform, the optic and the haptics are made of bio-compatible material with the required rigidity, flexibility and optical qualities. The ring platform has an over-all diameter of 11 to 14 mm for humans and it is varied for veterinary use depending on the species. The thickness of the ring platform is varied from 1mm to 2.5 mm. It is of hydro phobic or hydrophilic material. It is coated with or stored/loaded with medications such as steroids, antibiotics, anti- glaucoma drugs, anti-degenerative drugs, anti-mitotic agents, to reduce posterior capsular opacification, etc.

According to one embodiment herein, the size of the haptics is in the range of 0.5 mm - 3.5 mm. The shape can be of any stable one but the number of haptics has to be 4 or more to provide stability against rolling.

According to one embodiment herein, the optic sizes are varied from 5 mm to 10 mm or more, based on the mammalian eye. The optic design too, can be of any functional type. It is also applicable to aspheric, multifocal, accommodative, and all types of intraocular lenses.

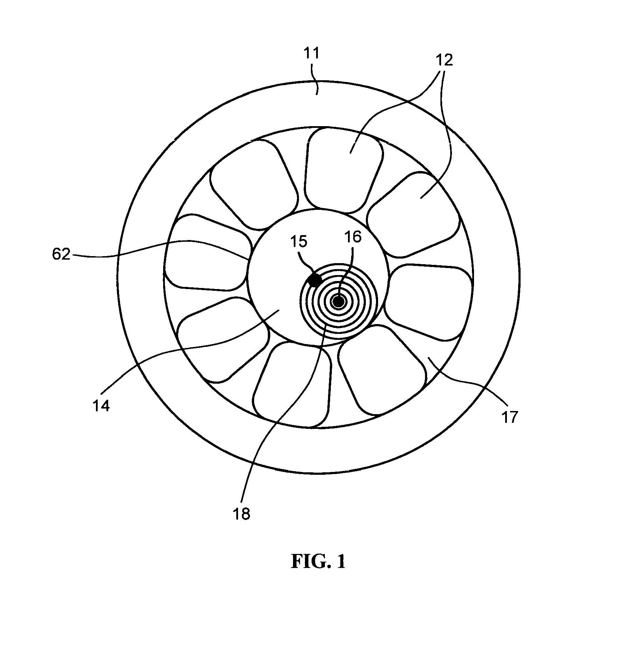

FIG. 1 illustrates the front view of the pupil- centered fovea-focused intraocular lens, according to one embodiment herein. A ring platform 11 is provided to be placed inside a capsular bag of a mammalian eye. An intraocular lens 19 is mounted on the ring platform 11. Plurality of haptics 12 are provided inside the ring platform 11 to exhibit improved pull strength and stability. The haptics 12 hold the intraocular lens 19 in its place. These haptics support structures 12 may be integrally formed with the intraocular lens 19 (as a one-piece lens) or separately manufactured and attached to the intraocular lens 19 (as a multi-piece lens). The intraocular lens 19 has a geometric centre 15 and an optical centre 16. The intraocular lens 19 has optical surfaces 14 and several refractive or diffractive zones 18. The distance between the geometric centre 15 and the optical centre 16 of the intraocular lens 19 is reduced and aligned with each other by decentering the optics. An empty space 17 exists between the adjacent haptics 12.

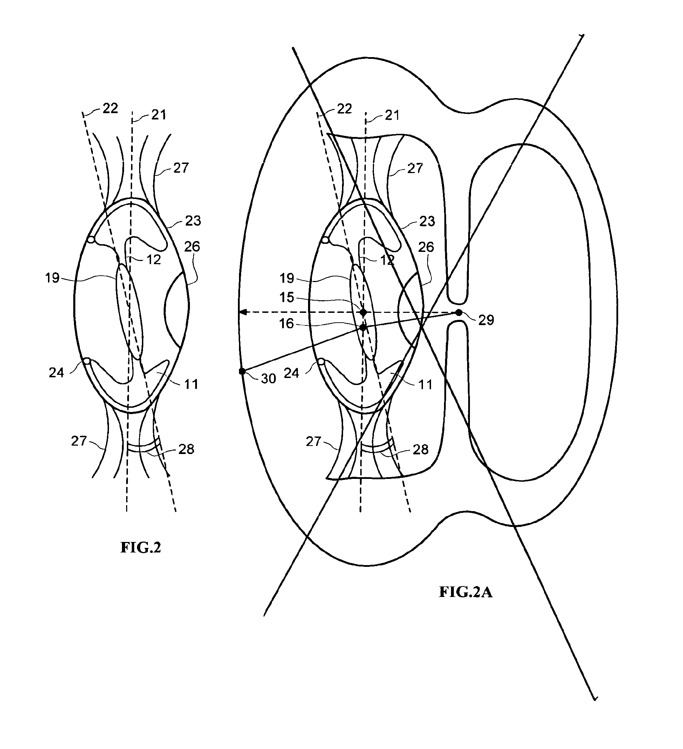

FIG. 2 illustrates the side view of pupil-centered fovea-focused intraocular lens, according to one embodiment herein. A capsular bag 23 is provided to hold the intraocular lens 19 and a BAG-based foundation ring called ring platform for supporting the intraocular lens 19. Capsulorhexis 26 is used to remove the lens capsule during cataract surgery. Zonules 27 are provided on both the poles. The zonules 27 are series of fibers that pass from the ciliary body to the capsular bag of the lens at or near its equator thereby holding the intraocular lens 19 in its position and enabling the ciliary muscles to act upon them. The intraocular lens 19 and zonules 27 form a diaphragm dividing the eye into a small anterior area, which contains aqueous humor and a larger posterior area which contains vitreous humor. The zonules 27 form a ring that is roughly triangular in a meridional section. The zonules 27 are made up of fibers that are transparent and straight for the most part. The tension of these fibers varies with the state of contraction of the ciliary muscle and thus affects the convexity of the lens. A ring of square edge 24 is arranged next to the intraocular lens, supporting bag based platform ring 11 on the both the poles. The plane of the capsular bag 21 and plane of the intraocular lens 22 are not same. The capsular bag 23 is tilted by an angle of tilt and turn 28 to focus and align to the fovea.

The various embodiments herein provide an innovative design of the intraocular lens so that the geometric centre of the lens is aligned to the visual axis, which lies in the pupil to correct an aberration. Further a support is provided to hold and tilt the intraocular lens to focus its axis towards the fovea. The intraocular lens of the embodiments herein overcomes the natural visual aberration and increases the sharpness in vision and improves visual quality.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the appended claims.

Although the embodiments herein are described with various specific embodiments, it will be obvious for a person skilled in the art to practice the embodiments herein with modifications. However, all such modifications are deemed to be within the scope of the claims.

## Claims

1. A pupil centered and fovea focused optics assembly for an implantable intraocular lens, the assembly comprising:
- a ring platform (11) provided inside a capsular bag (23) of a mammalian eye;
- an intraocular lens (19) with an optical center (16) and a geometric center (15) mounted on the ring platform (11),
the optical center (16) of the intraocular lens (19) is calculatedly decentered with respect to the geometric center (15) of the intraocular lens (19) to align the optical centre of the lens with a visual axis of a pupil of the mammalian eye to improve the visual quality and to prevent an aberration, and **characterised in that** a plane of the intraocular lens (19) is turned and tilted through a preset angle (28) to point an optic axis of the intraocular lens (19) to the Fovea to compensate for the decentered pupil.

2. The assembly according to claim 1, wherein the preset angle (28) is calculated based on an overall diameter of the capsular bag (23), a thickness of the ring platform (11), a decentering direction and a decentering distance of the optical axis of the intraocular lens (19).

3. The assembly according to claim 1, wherein the overall diameter of the capsular bag (23), the thickness of the ring platform (11), the decentering direction and the decentering distance of the optical axis of the intraocular lens (19) are calculated by performing a three dimensional scanning of the mammalian eye.

4. The assembly according to claim 1, wherein the intraocular lens (19) is mounted on the ring platform (11) through a plurality of haptics (12).

5. The assembly according to claim 4, wherein the plurality of haptics (12) hold the intraocular lens (19) in a desired place.

6. The assembly according to claim 5, wherein the ring platform (11) arranged 5 inside the capsular bag (23) supports the intraocular lens (19) through the haptics (12) and points the intraocular lens (19) towards a Fovea of the mammalian eye.

7. The assembly according to claim 6, wherein the haptics (12) of the intraocular lens (19) is fixed to a front part or a middle part or a back part of the ring platform (11).

8. The assembly according to claim 7, wherein an upper haptics of the intraocular lens (19) is fixed to the front part of the ring platform (11) and a lower haptics is fixed to the back part of the ring platform.

9. The assembly according to claim 1, wherein the ring platform (11) is provided with markings to identify a vertical meridian and a horizontal meridian.

10. The assembly according to claim 4, wherein the intraocular lens (19) and the haptics (12) are provided with markings, to identify a vertical meridian and a horizontal meridian.

11. The assembly according to claim 4, wherein the haptics (12) are integrally formed with the intraocular lens (19) as a one-piece lens or separately manufactured and attached to the intraocular lens (19).

12. The assembly according to claim 1, wherein the intraocular lens (19) has optical surfaces (14) and several refractive or diffractive zones (18).

## Patentansprüche

1. Pupillenzentrierte und Fovea-fokussierte Optikanordnung für eine implantierbare Intraokularlinse, wobei die Anordnung umfasst:
- eine Ringplattform (11), die innerhalb eines Kapselsacks (23) eines Säugerauges zur Verfügung gestellt wird;
- eine Intraokularlinse (19) mit einem optischen Zentrum (16) und einem geometrischen Zentrum (15), die auf der Ringplattform (11) befestigt ist,
wobei das optische Zentrum (16) der Intraokularlinse (19) in Bezug auf das geometrische Zentrum (15) der Intraokularlinse (19) einkalkuliert dezentriert ist, um das optische Zentrum der Linse mit einer Blickachse einer Pupille des Säugerauges zur Deckung zu bringen, um die Bildqualität zu verbessern und eine Aberration zu vermeiden, und
**dadurch gekennzeichnet, dass** eine Ebene der Intraokularlinse (19) um einen voreingestellten Winkel (28) gedreht und geneigt ist, um eine optische Achse der Intraokularlinse (19) auf die Fovea zu richten, um die dezentrierte Pupille auszugleichen.

2. Anordnung gemäß Anspruch 1, wobei der voreingestellte Winkel (28) basierend auf einem Gesamtdurchmesser des Kapselsacks (23), einer Dicke der Ringplattform (11), einer Dezentrierungsrichtung und einem Dezentrierungsabstand der optischen Achse der Intraokularlinse (19) berechnet wird.

3. Anordnung gemäß Anspruch 1, wobei der Gesamtdurchmesser des Kapselsacks (23), die Dicke der Ringplattform (11), die Dezentrierungsrichtung und der Dezentrierungsabstand der optischen Achse der Intraokularlinse (19) durch eine Durchführung einer dreidimensionalen Abtastung des Säugerauges berechnet werden.

4. Anordnung gemäß Anspruch 1, wobei die Intraokularlinse (19) durch eine Mehrzahl von haptischem Material (12) auf der Ringplattform (11) befestigt wird.

5. Anordnung gemäß Anspruch 4, wobei die Mehrzahl von haptischen Stoffen (12) die Intraokularlinse (19) an einer gewünschten Stelle halten.

6. Anordnung gemäß Anspruch 5, wobei die Ringplattform (11), die innerhalb des Kapselsacks (23) angeordnet ist, die Intraokularlinse (19) durch das haptische Material (12) unterstützt und die Intraokularlinse (19) auf eine Fovea des Säugerauges richtet.

7. Anordnung gemäß Anspruch 6, wobei das haptische Material (12) der Intraokularlinse (19) an einem Vorderteil oder einem Mittelteil oder einem Hinterteil der Ringplattform (11) befestigt ist.

8. Anordnung gemäß Anspruch 7, wobei ein oberes haptisches Material der Intraokularlinse (19) an dem Vorderteil der Ringplattform (11) befestigt ist und ein unteres haptisches Material an dem Hinterteil der Ringplattform befestigt ist.

9. Anordnung gemäß Anspruch 1, wobei die Ringplattform (11) mit Markierungen ausgestattet ist, um einen vertikalen Meridian und einen horizontalen Meridian zu identifizieren.

10. Anordnung gemäß Anspruch 4, wobei die Intraokularlinse (19) und das haptische Material (12) mit Markierungen ausgestattet sind, um einen vertikalen Meridian und einen horizontalen Meridian zu identifizieren.

11. Anordnung gemäß Anspruch 4, wobei das haptische Material (12) mit der Intraokularlinse (19) als eine einteilige Linse integral gebildet oder getrennt hergestellt und an der Intraokularlinse (19) befestigt wird.

12. Anordnung gemäß Anspruch 1, wobei die Intraokularlinse (19) über optische Oberflächen (14) und mehrere lichtbrechende oder lichtbeugende Bereiche (18) verfügt.

## Revendications

1. Ensemble optique centré sur la pupille et focalisé sur la fovéa pour un cristallin artificiel implantable, l'ensemble comprenant :
- une plateforme annulaire (11) placée à l'intérieur d'un sac capsulaire (23) d'un oeil de mammifère ;
- un cristallin artificiel (19) ayant un centre optique (16) et un centre géométrique (15) monté sur la plateforme annulaire (11),
le centre optique (16) du cristallin artificiel (19) est décentré de façon calculée par rapport au centre géométrique (15) du cristallin artificiel (19) pour aligner le centre optique du cristallin avec un axe visuel d'une pupille de l'oeil de mammifère pour améliorer la qualité visuelle et pour prévenir les aberrations, et
**caractérisé en ce que** l'on fait tourner et incliner un plan du cristallin artificiel (19) selon un angle préétabli (28) pour faire pointer un axe optique du cristallin artificiel (19) vers la fovéa afin de compenser le décentrage de la pupille.

2. Ensemble selon la revendication 1, dans lequel l'angle préétabli (28) est calculé d'après un diamètre hors tout du sac capsulaire (23), une épaisseur de la plateforme annulaire (11), une direction de décentrage et une distance de décentrage de l'axe optique du cristallin artificiel (19).

3. Ensemble selon la revendication 1, dans lequel le diamètre hors tout du sac capsulaire (23), l'épaisseur de la plateforme annulaire (11), la direction de décentrage et la distance de décentrage de l'axe optique du cristallin artificiel (19) sont calculés en réalisant un balayage tridimensionnel de l'oeil de mammifère.

4. Ensemble selon la revendication 1, dans lequel le cristallin artificiel (19) est monté sur la plateforme annulaire (11) à travers une pluralité d'éléments de toucher (12).

5. Ensemble selon la revendication 4, dans lequel les éléments de toucher (12) maintiennent le cristallin artificiel (19) en un endroit voulu.

6. Ensemble selon la revendication 5, dans lequel la plateforme annulaire (11) placée à l'intérieur du sac capsulaire (23) supporte le cristallin artificiel (19) à travers les éléments de toucher (12) et fait pointer le cristallin artificiel (19) vers une fovéa de l'oeil de mammifère.

7. Ensemble selon la revendication 6, dans lequel les éléments de toucher (12) du cristallin artificiel (19) sont fixés sur une partie avant ou une partie médiane ou une partie arrière de la plateforme annulaire (11).

8. Ensemble selon la revendication 7, dans lequel un élément de toucher supérieur du cristallin artificiel (19) est fixé sur la partie avant de la plateforme annulaire (11) et un élément de toucher inférieur est fixé sur la partie arrière de la plateforme annulaire.

9. Ensemble selon la revendication 1, dans lequel la plateforme annulaire (11) est pourvue de repères pour identifier un méridien vertical et un méridien horizontal.

10. Ensemble selon la revendication 4, dans lequel le cristallin artificiel (19) et les éléments de toucher (12) sont pourvus de repères, pour identifier un méridien vertical et un méridien horizontal.

11. Ensemble selon la revendication 4, dans lequel les éléments de toucher (12) sont formés d'un seul tenant avec le cristallin artificiel (19) en tant que cristallin en une pièce ou sont fabriqués séparément puis fixés sur le cristallin artificiel (19).

12. Ensemble selon la revendication 1, dans lequel le cristallin artificiel (19) a des surfaces optiques (14) et plusieurs zones de réfraction ou de diffraction (18).
